# EUROPÄISCHE PATENTSCHRIFT

(11) **EP 2 880 152 B1**
(45) Veröffentlichungstag und Bekanntmachung des Hinweises auf die Patenterteilung: **14.03.2018**
(21) Anmeldenummer: 13745085.4
(22) Anmeldetag: 02.08.2013
(51) Int. Cl.: C12N 5/095, G01N 33/50, C12N 5/09

(54) **VERFAHREN ZUR KULTIVIERUNG EINER SUBPOPULATION ZIRKULIERENDER EPITHELIALER TUMORZELLEN AUS EINER KÖRPERFLÜSSIGKEIT**
METHOD FOR CULTURING A SUBPOPULATION OF CIRCULATING EPITHELIAL TUMOUR CELLS FROM A BODY FLUID
PROCÉDÉ DE CULTURE D'UNE SOUS-POPULATION DE CELLULES TUMORALES ÉPITHÉLIALES CIRCULANTES PROVENANT D'UN FLUIDE CORPOREL

(30) Priorität: 03.08.2012 DE 102012213838
(43) Veröffentlichungstag der Anmeldung: 10.06.2015
(60) Teilanmeldung: 18152803.5
(73) Patentinhaber: Pachmann, Ulrich, 95448 Bayreuth (DE); Pachmann, Katharina, 95448 Bayreuth (DE)
(72) Erfinder: Pachmann, Ulrich, 95448 Bayreuth (DE); Pachmann, Katharina, 95448 Bayreuth (DE)
(74) Vertreter: Dr. Gassner & Partner mbB
(86) Internationale Anmeldenummer: PCT/EP2013/066325
(87) Internationale Veröffentlichungsnummer: WO 2014/020169

(56) Entgegenhaltungen:
- WO-A2-2007/079293
- DE-A1-102009 047 146
- NINGFANG WANG ET AL: "Detection of circulating tumor cells and tumor stem cells in patients with breast cancer by using flow cytometry", TUMOR BIOLOGY, Bd. 33, Nr. 2, 13. Januar 2012 (2012-01-13), Seiten 561-569, XP055081770, ISSN: 1010-4283, DOI: 10.1007/s13277-011-0303-1
- HEKIMIAN K ET AL: "Detection of tumor stem cells among circulating epithelial tumor cells (CETC) and relationship to therapy response", CANCER RESEARCH; 31ST ANNUAL SAN ANTONIO BREAST CANCER SYMPOSIUM; SAN ANTONIO, TX, USA; DECEMBER 10 -14, 2008, AMERICAN ASSOCIATION FOR CANCER RESEARCH, US, Bd. 69, Nr. 2 supplement S, 15. Januar 2009 (2009-01-15), Seite 1, XP008135237, ISSN: 0008-5472, DOI: 10.1158/0008-5472.SABCS-5053
- HIRSCHHAEUSER F ET AL: "Multicellular tumor spheroids: An underestimated tool is catching up again", JOURNAL OF BIOTECHNOLOGY, ELSEVIER SCIENCE PUBLISHERS, AMSTERDAM, NL, Bd. 148, Nr. 1, 1. Juli 2010 (2010-07-01), Seiten 3-15, XP027096372, ISSN: 0168-1656 [gefunden am 2010-01-25]
- Tie Chen ET AL: "Identification and expansion of cancer stem cells in tumor tissues and peripheral blood derived from gastric adenocarcinoma patients", Cell Research, vol. 22, no. 1, 5 July 2011 (2011-07-05), pages 248-258, XP055124283, ISSN: 1001-0602, DOI: 10.1038/cr.2011.109
- Su-Feng Chen ET AL: "Nonadhesive Culture System as a Model of Rapid Sphere Formation with Cancer Stem Cell Properties", PLoS ONE, vol. 7, no. 2, 16 February 2012 (2012-02-16), page e31864, XP055124222, ISSN: 1932-6203, DOI: 10.1371/journal.pone.0031864
- DONGLIANG CAO ET AL: "A New Tumorsphere Culture Condition Restores Potentials of Self-Renewal and Metastasis of Primary Neuroblastoma in a Mouse Neuroblastoma Model", PLOS ONE, vol. 9, no. 1, 22 January 2014 (2014-01-22), page e86813, XP055403773, DOI: 10.1371/journal.pone.0086813

## Beschreibung

Die Erfindung betrifft ein Verfahren zur Kultivierung einer Subpopulation zirkulierender epithelialer Tumorzellen aus einer Körperflüssigkeit eines Menschen oder Tiers. Bei dem Tier kann es sich um ein Säugetier handeln.

Aus Dontu, G. et al., Genes & Development 17, 2003, Seiten 1253 bis 1270 ist ein Verfahren zur in-vitro-Kultivierung humaner epithelialer Zellen aus Brustgewebe bekannt. Zur Erzeugung der Zellen wurden Einzelzellsuspensionen humaner Mammaepithelzellen durch mechanische und enzymatische Dissoziation von Brustgewebe hergestellt und unter Bedingungen, die keine Adhäsion an ein Substrat erlauben, kultiviert. Dabei proliferierte nur eine kleine Population der Zellen in Suspension als so genannte Mammosphären.

Aus Lu, J. et al., Int. J. Cancer 126, 2010, Seiten 669 bis 683 ist es bekannt, Zellen aus peripherem Blut von Brustkrebspatientinnen zunächst mittels einer Ficoll-Dichtegradientenzentrifugation anzureichern und die dadurch erhaltenen mononukleären Zellen auf einer Kollagen-Adhäsionsmatrix auszusäen und für 12 Stunden in einem Zellkulturmedium in einem CO₂-Zellinkubator zu inkubieren. Nur Zellen, die in dieser Zeit adhärieren konnten, werden anschließend weiter kultiviert, während nicht-adhärente Zellen und altes Medium entfernt werden. Zur Kultivierung der adhärenten Zellen wird frisches Zellkulturmedium zugegeben, welches dann alle 3 Tage durch frisches Zellkulturmedium ersetzt wird. Die Anzahl und Größe der Zellen nahm dabei mit der Kultivierungszeit zu.

Nach 20 Tagen fanden sich große ausgebreitete Zellen mit epithelialer Morphologie.

Aus Chen, T. et al., Cell Research (2012), Band 22, Seiten 248 - 258 ist es bekannt, Krebsstammzellen aus dem Blut von Patienten mit Magenadenokarzinom zu isolieren und daraus in einem serumfreien Medium Tumorsphären zu züchten. Diese sind in der Lage, in Nacktmäusen Tumore zu induzieren, während in serumhaltigem Medium kultivierte Sphären-Zellen keine detektierbare Tumore in Mäusen erzeugten.

Die DE 10 2009 047 146 A1 offenbart ein Verfahren zum Vorhersagen des Ansprechens einer von einem soliden epithelialen Tumor verursachten Tumorerkrankung eines Patienten auf eine therapeutische Maßnahme. Dabei werden epitheliale Tumorzellen aus einer Körperflüssigkeit des Patienten jeweils in einem Zellkulturmedium aufgenommen. Das Zellkulturmedium enthält bevorzugt keinen zugesetzten Wachstumsfaktor und kein zugesetztes Wachstumsfaktoren enthaltendes Serum. Tumorzellen aus einer Probe des die Tumorzellen enthaltenden Zellkulturmediums werden der therapeutischen Maßnahme ausgesetzt, während die Tumorzellen aus einer ebensolchen Kontrollprobe des die Tumorzellen enthaltenden Zellkulturmediums unbehandelt bleiben. Anschließend wird für die Probe und die Kontrollprobe jeweils der Anteil absterbender und toter epithelialer Tumorzellen an der Gesamtzahl der epithelialen Tumorzellen und daraus eine durch die therapeutische Maßnahme bedingte Absterberate der epithelialen Tumorzellen als Maß für das Ansprechen bestimmt. Zwischen der therapeutischen Maßnahme und dem Bestimmen des Anteils absterbender und toter epithelialer Tumorzellen an der Gesamtzahl der epithelialen Tumorzellen kann eine Zeit von einigen Stunden bis Tagen liegen. In dieser Zeit werden die Tumorzellen unter üblichen Zellkulturbedingungen gehalten, welche ohne therapeutische Maßnahme ein Überleben der Tumorzellen im Zellkulturmedium erlauben. Es ist nicht offenbart, dass unter diesen Bedingungen eine Proliferation der Tumorzellen erfolgt.

Aufgabe der vorliegenden Erfindung ist es, ein alternatives Verfahren zur Kultivierung von Zellen aus einer Körperflüssigkeit von Patienten/Patientinnen mit einer auf einen epithelialen Tumor zurückgehenden Tumorerkrankung anzugeben. Die kultivierten Zellen sollen eine Charakterisierung der Tumorerkrankung erlauben.

Die Aufgabe wird durch die Merkmale des Patentanspruchs 1 gelöst. Zweckmäßige Ausgestaltungen ergeben sich aus den Merkmalen der Patentansprüche 2 bis 6.

Erfindungsgemäß ist in Verfahren zur Kultivierung zirkulierender epithelialer Tumorzellen aus einer Körperflüssigkeit eines an einem epithelialen Tumor erkrankten Menschen oder Tiers, insbesondere Säugetiers, vorgesehen. Dabei werden in der Körperflüssigkeit enthaltene je mindestens einen Zellkern enthaltende Zellen von der Körperflüssigkeit abgesondert und über mindestens 24 Stunden, insbesondere mindestens 2 Tage, insbesondere mindestens 3 Tage, insbesondere mindestens 4 Tage, insbesondere mindestens 5 Tage, insbesondere mindestens 6 Tage, insbesondere mindestens 7 Tage, in Suspension, d. h. unter Vermeidung einer Zelladhäsion an ein Substrat, unter Bildung von Sphäroiden kultiviert. Bei der Kultivierung unter Bildung von Sphäroiden werden die Zellen mindestens solange kultiviert, bis sie durch Proliferation unter Zusatz mindestens des Wachstumsfaktors EGF Sphäroide gebildet haben. Unter epithelialen Tumorzellen werden hier insbesondere Tumorzellen verstanden, die das Epitheliale Zelladhäsionsmolekül (EpCAM, CD326) oder das entsprechende tierische Molekül tragen. Es kann sich dabei beispielsweise um Zellen von Karzinomen oder Sarkomen handeln.

Keinen Zellkern enthaltende Zellen sind im Allgemeinen gegebenenfalls in der Körperflüssigkeit enthaltene Erythrozyten. Diese können auch in den abgesonderten Zellen enthalten sein. Wenn in der Körperflüssigkeit intakte Erythrozyten enthalten sind, können die je mindestens einen Zellkern enthaltenden Zellen von den intakten Erythrozyten, insbesondere durch Lysieren der intakten Erythrozyten, abgesondert werden. Dies kann vor, bei oder nach dem Absondern der je mindestens einen Zellkern enthaltenden Zellen von der Körperflüssigkeit erfolgen.

Die Kultivierung in Suspension ist eine Kultivierung unter Bedingungen, die keine Adhäsion an ein Substrat erlauben. Eine solche Kultivierung kann dadurch erfolgen, dass zur Kultivierung kein mit Kollagen beschichtetes oder auf andere Weise eine Zelladhäsion förderndes Zellkulturgefäß verwendet wird und/oder eine die Tumorzellen enthaltende Suspension in regelmäßigen Abständen oder ständig bewegt wird. Ein eine Zelladhäsion förderndes Zellkulturgefäß kann beispielsweise aus einem oberflächlich Ladungen tragenden Kunststoff bestehen. Eine Zelladhäsion kann auch dadurch vermieden werden, dass das Zellkulturgefäß ein mit einem Silikon oder einem Silan beschichtetes Zellkulturgefäß ist.

Im Gegensatz zu der aus Lu et al. bekannten Kultivierung wird bei der Kultivierung in Suspension eine Proliferation einer Subpopulation von in der Körperflüssigkeit enthaltenen zirkulierenden epithelialen Tumorzellen induziert oder ermöglicht, die zur Proliferation keine Adhärenz an einer Oberfläche benötigen.

Gemäß Lu, J. et al., Seite 671, rechte Spalte, 2. Absatz werden nicht-adhärente Zellen nach 12 Stunden entfernt und somit nicht weiterkultiviert. Spezifisch adhärent an einer Oberfläche wachsende zirkulierende epitheliale Tumorzellen werden also an einer beschichteten Oberfläche eines Zellkulturgefäßes kultiviert und durch die Adhäsion an dieser Oberfläche selektiert. Die Erfinder des vorliegenden Verfahrens haben jedoch erkannt, dass die zirkulierenden epithelialen Tumorzellen nicht nur adhärent wachsende Zellen, sondern auch solche Zellen umfassen, die ohne Adhäsion klonal proliferieren können. Sie haben weiterhin erkannt, dass gerade die Kultivierung dieser nicht adhärent wachsenden Zellen zu Zellklonen führt, deren Zellen für die Tumorerkrankung charakteristisch sind und damit eine gute Charakterisierung der Tumorerkrankung, selbst nach Entfernung eines der Tumorerkrankung zu Grunde liegenden Primärtumors, erlauben. Bei der Tumorerkrankung kann es sich um ein Mamma-, Prostata-, Lungen-, Nieren-, Leber-, Pankreas- oder Kolonkarzinom handeln.

Es hat sich gezeigt, dass den gesamten zirkulierenden Tumorzellen für eine Überwachung einer Antitumor-Therapie insbesondere dann eine wichtige klinische Bedeutung zukommt, wenn sich noch keine nachweisbaren Metastasen gebildet haben. Das Ansprechen der zirkulierenden Tumorzellen auf eine Therapie korreliert stark mit einem rezidivfreien Überleben. Von einer großen Zahl von aus einem Tumorgewebe eines epithelialen Tumors in den Blutkreislauf abgegebenen Tumorzellen ist aber nur ein Bruchteil dazu befähigt, im sekundären Gewebe Metastasen zu bilden.

Die Erfinder des erfindungsgemäßen Verfahrens haben weiterhin erkannt, dass es sich bei der Subpopulation der zirkulierenden Tumorzellen, die ohne Adhäsion proliferieren können, um sogenannte Tumorstammzellen oder tumorinitiierende Zellen handelt. Sie haben auch erkannt, dass diese Zellen in Form von Sphäroiden wachsen, d. h. beim Proliferieren eine kugelartige dreidimensionale Struktur ausbilden können. Die Kultivierung der epithelialen Tumorzellen in Suspension erlaubt die Bildung solcher Sphäroide und dadurch einen Nachweis des Vorhandenseins von Tumorstammzellen als Subpopulation in der Population der zirkulierenden Tumorzellen. In diesen Sphäroiden bilden die Stammzellen Progenitorzellen in unterschiedlichen Differenzierungsstadien. Es war bisher nicht bekannt, dass zirkulierende epitheliale Tumorzellen Tumorstammzellen umfassen, die sich in Form von Sphäroiden kultivieren lassen. Es wird angenommen, dass diese Tumorstammzellen für eine Metastasierung von entscheidender Bedeutung sind.

Bei der Körperflüssigkeit kann es sich um Lymphe oder Blut, insbesondere peripheres Blut, handeln. Peripheres Blut weist dabei den Vorteil der guten Zugänglichkeit und leichten Verfügbarkeit auf.

Bei dem erfindungsgemäßen Verfahren werden die in der Körperflüssigkeit enthaltenen je mindestens einen Zellkern enthaltenden Zellen von der Körperflüssigkeit ohne Selektion bestimmter dieser Zellen abgesondert, in ein Zellkulturmedium überführt und unter Zellkulturbedingungen in einem Zellkulturmedium gehalten. Die Zellkulturbedingungen sind dabei im Allgemeinen solche, die zur Kultivierung epithelialer Zellen geeignet sind. Das Zellkulturmedium enthält im Allgemeinen ein tierisches Serum, wie z. B. fötales Kälberserum, L-Glutamin, einen Wachstumsstimulator, wie z. B. Insulin, Hydrocortison und den Wachstumsfaktor EGF, in einem Kulturmedium, wie beispielsweise RPMI 1640, Dulbecco's modifiziertem Eagle's Medium (DMEM) oder einer Mischung aus DMEM und RPMI 1640. Die Zellkulturbedingungen umfassen im Allgemeinen eine Temperatur im Bereich von 35,5 °C bis 37,5 °C, insbesondere eine Temperatur im Bereich von 36,8 °C bis 37,1 °C, insbesondere eine Temperatur von 37 °C, und eine 4,5% bis 5,5% CO₂, insbesondere 5% CO₂, enthaltende Atmosphäre. Für epitheliale Zellen geeignete Zellkulturbedingungen und ein für epitheliale Zellen geeignetes Zellkulturmedium sind dem Fachmann auf dem Gebiet der Kultivierung von Säugerzellen, beispielsweise aus Lu et al., bekannt. Gegebenenfalls erforderliche Anpassungen an die jeweiligen Tumorzellen, etwa bei der Wahl des zu Grunde liegenden Kulturmediums oder dem Anteil an fötalem Kälberserum, liegen im Rahmen des fachmännischen Könnens und erfordern keinen unzumutbaren experimentellen Aufwand.

Die Kultivierung der aus der Körperflüssigkeit ohne Selektion bestimmter Zellen abgesonderten Zellen in Suspension bewirkt, dass zwar eine Vielzahl unterschiedlicher Zellen, darunter beispielsweise auch Leukozyten, in das Zellkulturmedium überführt werden, davon aber nur solche Zellen proliferieren, die dazu in Suspension in der Lage sind. Insofern bewirkt die Kultivierung eine spezifische Vermehrung genau dieser Zellen. Durch die Absonderung der Zellen aus der Körperflüssigkeit ohne Selektion bestimmter Zellen wird vermieden, dass die Zellen, die in Suspension proliferieren können und oft nur einen geringen Anteil der zirkulierenden epithelialen Tumorzellen bilden, verloren gehen.

Dem Zellkulturmedium kann mehrfach oder regelmäßig, beispielsweise alle 5 Tage, frisches Zellkulturmedium zugesetzt werden. Alternativ können die Zellen mehrfach oder regelmäßig in frisches Zellkulturmedium umgesetzt werden. Dazu können die Zellen vorsichtig, das heißt bei verhältnismäßig niedrigen Drehzahlen abzentrifugiert und in frischem Zellkulturmedium resuspendiert werden.

Bei einer Ausgestaltung des Verfahrens werden von den kultivierten Tumorzellen, insbesondere für eine weitere Kultivierung, eine Analyse oder einen Test auf deren Sensitivität gegenüber einer therapeutischen Maßnahme, solche Tumorzellen abgesondert, die bei der Kultivierung Sphäroide gebildet haben. Die von Tumorzellen gebildeten Sphäroide werden im Folgenden auch Tumorsphäroide genannt. Die Absonderung kann aufgrund der größeren Maße der Tumorsphäroide im Vergleich zu Einzelzellen einfach, beispielsweise durch Zentrifugieren mit niedriger Drehzahl oder durch Absetzen lassen oder durch Einsaugen der Tumorsphäroide in eine Kapillare unter Beobachtung mittels eines Mikroskops, erfolgen.

Die erfindungsgemäß kultivierten und in Sphäroiden vorliegenden Tumorzellen können zur Untersuchung einer Selbsterneuerungsfähigkeit der in den Sphäroiden enthaltenen Tumorzellen und/oder von in den Sphäroiden erzeugten Vorläuferzellen und/oder zum Testen der Sensitivität der Tumorzellen in den Sphäroiden gegenüber einem Medikament oder einer therapeutischen Maßnahme zur Behandlung einer dem Vorkommen der zirkulierenden epithelialen Tumorzellen in dem Menschen oder Tier, insbesondere Säugetier, zu Grunde liegenden Tumorerkrankung verwendet werden. Die Erfinder haben erkannt, dass die in den Sphäroiden vorliegenden Tumorzellen den Zellen entsprechen, die im Menschen oder Tier ein Tumorwachstum, selbst nach vollständiger Resektion eines soliden epithelialen Primärtumors, in Gang halten, indem sie eine Basis für die Bildung von Metastasen bilden. Mittels dieser Tumorzellen lässt sich die Tumorerkrankung daher besonders gut charakterisieren. Die auch in den Sphäroiden auftretende Selbsterneuerungsfähigkeit und Erzeugung von Vorläuferzellen ist ein besonderes Kennzeichen von Tumorstammzellen. Das Testen der Sensitivität der die Sphäroide bildenden Zellen gegenüber einem Medikament oder einer therapeutischen Maßnahme zur Behandlung einer dem Vorkommen der zirkulierenden epithelialen Tumorzellen in dem Menschen oder Tier zu Grunde liegenden Tumorerkrankung erlaubt eine gute, völlig neuartige Vorhersage über die Effizienz des Medikaments oder der therapeutischen Maßnahme bei einer Verhinderung einer Metastasenbildung bei der Tumorerkrankung.

Bei der therapeutischen Maßnahme kann es sich um eine physikalische Maßnahme, insbesondere eine Bestrahlung oder Hyperthermiebehandlung, oder eine pharmazeutische Maßnahme, insbesondere eine Chemotherapie, eine Hormonbehandlung oder eine Behandlung mit Antikörpern, handeln. Weiterhin können an den Tumorsphäroiden neue und bekannte Medikamente auf ihre Wirksamkeit hin getestet werden.

Die in den Sphäroiden enthaltenen Tumorzellen können mittels gegen das humane epitheliale Antigen EpCAM (Epithelial cell adhesion molecule) gerichtete Antikörper fluoreszensmarkiert und anschließend mittels eines Bildanalyseverfahrens, z. B. mittels Laserstrahlung, analysiert werden. Eine Vereinzelung der Tumorzellen ist dazu nicht erforderlich. Die Antikörper erlauben eine Charakterisierung der Zellen als epitheliale Zellen. Zusätzlich können die in den Sphäroiden enthaltenen Tumorzellen mit einem durch Fluoreszenz detektierbaren spezifisch oder bevorzugt tote Zellen färbenden Stoff, insbesondere Propidiumiodid, gefärbt werden. Das erlaubt das Erkennen von toten Zellen, wie sie beispielsweise häufig im Inneren der Sphäroide vorkommen. Zur Bildanalyse kann beispielsweise ein Laser Scanning Cytometer oder ein Fluoreszenzmikroskop, insbesondere mit einer Bilderfassungseinheit/Kamera und einer Analysesoftware, wie z. B. die Olympus SCAN^{R} Screening-Station, verwendet werden.

Das Markieren, Vermessen, und/oder Färben kann in Gegenwart eines Ca²⁺-Chelators, insbesondere EDTA oder EGTA, erfolgen. Durch den Ca²⁺-Chelator kann ein Verklumpen der Sphäroide verhindert werden.

Sphäroide bildende Tumorzellen, die nach einem erfindungsgemäßen Verfahren kultiviert wurden, können zur immunologischen Behandlung einer Tumorerkrankung eines Menschen oder Tiers, insbesondere Säugetiers, verwendet werden. Dazu können die in den Sphäroiden enthaltenen Tumorzellen, beispielsweise mittels Strahlung, abgetötet oder zumindest so behandelt werden, dass sie nicht mehr proliferieren können. Anschließend können die Tumorzellen in einem Adjuvans suspendiert und anschließend dem Menschen oder Tier, beispielsweise subkutan, verabreicht werden. Dadurch wird die Bildung von Antikörpern gegen die in Form von Sphäroiden wachsenden Tumorzellen in dem Menschen oder Tier ausgelöst. Die dadurch gebildeten Antikörper können dann auch die sonstigen Tumorzellen in dem Menschen oder Tier angreifen und damit zur Rückbildung des Tumors beitragen.

Nachfolgend wird die Erfindung anhand eines Ausführungsbeispiels und der Figuren näher beschrieben. Es zeigen:
- Fig. 1a, b, c: Sphäroide von Tumorzellen aus peripherem Blut eines Patienten mit Kolonkarzinom nach 7 Tagen (Fig. 1a), 14 Tagen (Fig. 1b) und 21 Tagen (Fig. 1c) Kultivierung und
- Fig. 2: eine fluoreszensmikroskopische Aufnahme einer mit Antikörpern und Propidiumiodid fluoreszenzgefärbten Tumorsphäroide.

Für die Durchführung der nachfolgend beschriebenen Verfahren wurden die folgenden Chemikalien, Reagenzien, Puffer, Lösungen, Antikörper, Geräte und Verbrauchsmaterialien sowie das angegebene Medium verwendet:

### Chemikalien und Reagenzien

| **Chemikalien und Reagenzien** | **Firma** |
|---|---|
| Ammoniumchlorid (NH₄Cl) (155 mM) | Sigma-Aldrich, USA |
| Kaliumhydrogencarbonat (KHCO₃) (10 mM) | Sigma-Aldrich, USA |
| Ethylendiamintetraacetat (EDTA) (0,5 M) | Sigma-Aldrich, USA |
| Dulbecco's Phosphate Buffered Saline (D-PBS) | GIBCO, USA |
| Flow-Check 770 Fluorospheres | Beckman Coullter, Irland |
| Propidium iodide solution (1 mg) | Sigma-Aldrich, USA |
| RPMI 1640 (1X) 500 ml | Invitrogen GmbH, Deutschland |
| Hepes buffer solution (1 M) | Invitrogen GmbH, Deutschland |
| Penicillin/Streptomycin (10000 U/ 10000 µg) | Invitrogen GmbH, Deutschland |
| L-Glutamin (200 mM) | Biochrom AG, Deutschland |
| Fetal bovine serum (FBS) | Invitrogen GmbH, Deutschland |
| Insulin (100 U/mL) | Sanofi-Aventis, USA |
| Hydrocortison 100 mg | Pfizer, USA |
| EGF | Sigma-Aldrich, USA |

### Puffer und Lösungen

| **Puffer und Lösungen** | **Bestandteile** | **Menge** |
|---|---|---|
| D-PBS | CaCl₂ | 0,901 mM |
| | MgCl₂-6H₂O | 0,493 mM |
| | KCl | 2,67 mM |
| | KH₂PO₄ | 1,47 mM |
| | NaCl | 137,93 mM |
| | Na₂HPO₄-7H₂O | 8,06 mM |
| D-PBS-EDTA | D-PBS | 500 ml |
| | EDTA | 2 ml |
| Erylysepuffer (in 1 L Aqua dest. lösen) | NH₄Cl | 8,3 g |
| | KHCO₃ | 1 g |
| | EDTA | 2 ml |
| Propidiumiodid Lösung (in 1 ml Aqua dest.) | PI | 3,5 µl |

### Antikörper

| **Antikörper** | **Firma** | **Artikelnummer** |
|---|---|---|
| CD 326 (EpCAM) mit fluoresceinisothiocyanat (FITC) konjugiert | Miltenyi Biotec GmbH, Deutschland | 130-080-301 |

### Geräte

| **Geräte** | **Firma** |
|---|---|
| Zentrifuge 5810 R | Eppendorf, Deutschland |
| Laser-Scanning-Mikroskop iCys™ | CompuCyte Corporation, USA |
| Vortexer | Bender und Hobein GmbH, Deutschland |

### Verbrauchsmaterialien

| **Verbrauchsmaterialien** | **Firma** |
|---|---|
| 15 ml Falconröhrchen | Labor Schubert, Deutschland |
| 1,5 ml Eppendorfhütchen | Labor Schubert, Deutschland |
| Mikropipetten | Eppendorf, Deutschland |
| Einweg-Pasteurpipetten, graduiert (3,2 ml) | ROTH, Deutschland |
| Mikrotiterplatten (MTP) mit Glasboden | Greiner bio-one, Deutschland |
| Kulturflaschen (25 cm², 65 ml) | neoLab, Deutschland |

### Zusammensetzung des Mediums

| **Chemikalien** | **Endkonzentration** |
|---|---|
| ein Kulturmedium vorzugsweise RPMI 1640 | |
| ein Serumzusatz vorzugsweise fötales Kälberserum | 5 % |
| L-Glutamin | 4 mM |
| Hepes | 15 mM |
| Wachstumsstimulator vorzugsweise Insulin | 5 µg/ml |
| Hydrocortison | 0,5 µg/ml |
| Antibiotika vorzugsweise Pen/Strep | 100 U/ml; 100 µg/ml |
| Wachstumsfaktoren vorzugsweise EGF | 40 ng/ml |

Blutproben wurden peripher-venös in 2-7 ml EDTA als Antikoagulans enthaltenden Röhrchen entnommen. Die Vitalität der zirkulierenden epithelialen Tumorzellen betrug im Durchschnitt 95%.

In einem Probenröhrchen wurde 1 ml der jeweiligen Blutprobe mit dem Erylysepuffer auf 15 ml Gesamtvolumen aufgefüllt und für 15 min bei einer Temperatur von 4 °C im Kühlschrank inkubiert. Als nächstes wurde die Probe bei 2.000 rpm für 7 min und einer Temperatur von 18 °C zentrifugiert. Der Überstand wurde im Folgeschritt abgekippt. Danach wurde das Pellet mit 2 ml Medium resuspendiert und in vorgelegte 3 ml Medium in eine Kulturflasche überführt (abhängig von der Leukozytenzahl: 5 ml Medium pro 10 000 Leukozyten). Diese wurde für 21 Tage bei 5% CO₂ und 37 °C inkubiert. Alle 5 Tage wurden 2 ml frisches Medium zugegeben. Alle 7 Tage d. h. am 7., 14., 21. und 28. Tag wurden die Tumorsphäroide unter dem Mikroskop begutachtet. Für die Analyse wurden die Tumorsphäroide durch eine sanfte Zentrifugation gesammelt und anschließend das Pellet mit 500 µl D-PBS-EDTA resuspendiert. 50 µl dieses Gemisches wurden im Anschluss in ein 1,5 ml Eppendorfhütchen überführt. Nachfolgend wurden 5 µl eines (FITC)-monoklonalen Antikörpers gegen das humane epitheliale Antigen (EpCAM) dazu pipettiert. Es folgte ein erneutes 15-minütiges Kühlen bei 4 °C. Zuletzt wurden 430 µl D-PBS-EDTA hinzugegeben und die nun fertigen Proben über Nacht bei 4 °C im Kühlschrank gelagert.

Am darauffolgenden Tag wurden jeweils 100 µl Zellsuspension der zu messenden Probe und 5 µl Propidiumiodid (PI) in eine Vertiefung einer ELISA-Platte pipettiert. Die Platte wurde dann abgedeckt und ca. 20 min ruhen gelassen, damit sich die Zellen am Boden absetzen konnten. Anschließend wurden die Zellen mit dem Laser Scanning Cytometer vermessen und die Messergebnisse ausgewertet.

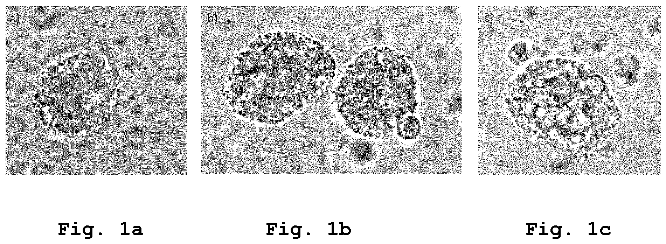

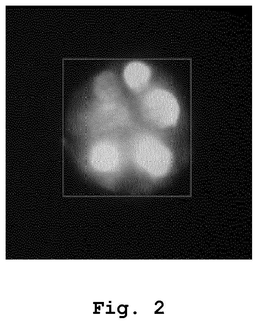

Es wurden nicht adhärente, dreidimensionale Sphäroide erhalten, die typisch für die Anwesenheit von Tumorstammzellen sind. Fig. 1a zeigt die Sphäroide nach 7 Tagen, Fig. 1b nach 14 Tagen und Fig. 1c nach 21 Tagen Kultivierung. Abbildung 2 zeigt eine rot gefärbte nekrotische Tumorzelle in der Mitte einer Zellgesamtheit einer Tumorsphäroide. Das Absterben dieser Tumorzelle ist möglicherweise auf eine fehlende Diffusion von Wachstumsfaktoren durch die diese Zelle umgebende dichte Zellpackung zurückzuführen.

Es konnte eine Subpopulation der zirkulierenden epithelialen Tumorzellen kultiviert werden, die Sphäroide bildet und eine hohe Proliferationskapazität besitzt.

## Patentansprüche

1. Verfahren zur Kultivierung zirkulierender epithelialer Tumorzellen aus einer Körperflüssigkeit eines an einem epithelialen Tumor erkrankten Menschen oder Tiers, wobei in der Körperflüssigkeit enthaltene je mindestens einen Zellkern enthaltende Zellen von der Körperflüssigkeit ohne Selektion bestimmter dieser Zellen abgesondert, in ein Zellkulturmedium überführt und unter Zellkulturbedingungen in einem Zellkulturmedium unter Zusatz mindestens eines Wachstumsfaktors gehalten werden, wobei die Zellen über mindestens 24 Stunden in Suspension mindestens solange kultiviert werden, bis eine zur Proliferation keine Adhärenz an einer Oberfläche benötigende Subpopulation der Tumorzellen durch Proliferation Sphäroide gebildet hat, wobei der Wachstumsfaktor EGF ist, wobei das Verfahren keine chirurgische Behandlung des menschlichen oder tierischen Körpers umfasst.

2. Verfahren nach Anspruch 1, wobei die Körperflüssigkeit Lymphe oder Blut, insbesondere peripheres Blut, ist.

3. Verfahren nach einem der vorhergehenden Ansprüche, wobei die Körperflüssigkeit intakte Erythrozyten enthält und die je mindestens einen Zellkern enthaltenden Zellen von den intakten Erythrozyten, insbesondere durch Lysieren der intakten Erythrozyten, abgesondert werden.

4. Verfahren nach einem der vorhergehenden Ansprüche, wobei die Zellkulturbedingungen eine Temperatur im Bereich von 35,5 °C bis 37,5 °C, insbesondere eine Temperatur im Bereich von 36,8 °C bis 37,1 °C, insbesondere eine Temperatur von 37 °C, und eine 4,5% bis 5,5% CO₂, insbesondere 5% CO₂, enthaltende Atmosphäre umfassen.

5. Verfahren nach einem der vorhergehenden Ansprüche, wobei dem Zellkulturmedium mehrfach oder regelmäßig frisches Zellkulturmedium zugesetzt wird oder wobei die Zellen mehrfach oder regelmäßig in frisches Zellkulturmedium umgesetzt werden.

6. Verfahren nach einem der vorhergehenden Ansprüche, wobei von den kultivierten Tumorzellen solche Tumorzellen abgesondert werden, die bei der Kultivierung die Sphäroide gebildet haben, indem die bei der Kultivierung gebildeten Sphäroide abgesondert werden.

## Claims

1. A method for culturing circulating epithelial tumor cells from a body fluid from a human or animal affected by an epithelial tumor, wherein cells present in the body fluid and containing at least one nucleus in each case are separated from the body fluid without selection of certain of these cells, transferred to a cell culture medium and kept under cell culture conditions in a cell culture medium with addition of at least one growth factor, wherein the cells are cultured for at least 24 hours in suspension at least until a subpopulation of the tumor cells which does not require adherence to a surface for proliferation has formed spheroids by proliferation, wherein the growth factor is EGF, wherein the method does not comprise any treatment of the human or animal body by surgery.

2. The method as claimed in claim 1, wherein the body fluid is lymph or blood, more particularly peripheral blood.

3. The method as claimed in either of the preceding claims, wherein the body fluid contains intact erythrocytes and the cells containing at least one nucleus in each case are separated from the intact erythrocytes, more particularly by lysing the intact erythrocytes.

4. The method as claimed in any of the preceding claims, wherein the cell culture conditions comprise a temperature within the range of 35.5°C to 37.5°C, more particularly a temperature within the range of 36.8°C to 37.1°C, more particularly a temperature of 37°C, and an atmosphere containing 4.5% to 5.5% CO₂, more particularly 5% CO₂.

5. The method as claimed in any of the preceding claims, wherein fresh cell culture medium is added to the cell culture medium repeatedly or regularly or wherein the cells are transferred to fresh cell culture medium repeatedly or regularly.

6. The method as claimed in any of the preceding claims, wherein tumor cells which have formed the spheroids during culturing are separated from the cultured tumor cells, by separating the spheroids formed during culturing.

## Revendications

1. Procédé de mise en culture de cellules tumorales épithéliales circulantes provenant d'un fluide corporel d'un être humain ou animal souffrant d'une tumeur épithéliale, dans lequel des cellules contenues dans le fluide corporel, contenant chacune au moins un noyau cellulaire sont séparées du fluide corporel sans sélection de certaines de ces cellules, transférées dans un milieu de culture cellulaire et maintenues dans des conditions de culture cellulaire dans un milieu de culture cellulaire avec ajout d'au moins un facteur de croissance, dans lequel les cellules sont mises en culture sur au moins 24 heures en suspension au moins jusqu'à ce qu'une sous-population des cellules tumorales ne nécessitant pour la prolifération aucune adhérence à une surface se soit formée par prolifération de sphéroïdes, dans lequel le facteur de croissance est le facteur de croissance épidermique (EGF), dans lequel le procédé ne comprend aucun traitement chirurgical du corps humain ou animal.

2. Procédé selon la revendication 1, dans lequel le fluide corporel est de la lymphe ou du sang, notamment du sang périphérique.

3. Procédé selon une des revendications précédentes, dans lequel le fluide corporel contient des érythrocytes intacts et les cellules contenant chacune au moins un noyau cellulaire sont séparées des érythrocytes intacts, notamment par lyse des érythrocytes intacts.

4. Procédé selon une des revendications précédentes, dans lequel les conditions de culture cellulaire comprennent une température dans la région de 35,5°C à 37,5°C, notamment une température dans la région de 36,8°C à 37,1 °C, notamment une température de 37°C, et une atmosphère contenant 4,5 % à 5,5 % de CO₂, notamment 5 % de CO₂.

5. Procédé selon une des revendications précédentes, dans lequel du milieu de culture cellulaire frais est ajouté plusieurs fois ou régulièrement au milieu de culture cellulaire ou dans lequel les cellules sont déplacées plusieurs fois ou régulièrement dans du milieu de culture cellulaire frais.

6. Procédé selon une des revendications précédentes, dans lequel des cellules tumorales qui ont formé les sphéroïdes lors de la mise en culture sont séparées des cellules tumorales mises en culture en ce que les sphéroïdes formés lors de la mise en culture sont séparés.
